# EUROPEAN PATENT APPLICATION

(11) **EP 0 897 002 A2**
(43) Date of publication of application: **17.02.1999**
(21) Application number: 98305494.1
(22) Date of filing: 10.07.1998
(51) Int. Cl.: C12N 15/12, C07K 14/47, C07K 16/18, A61K 38/17, C12Q 1/68, G01N 33/68

(54) **U62317, a protein having a JNK-binding domain**

(30) Priority: 14.08.1997 EP 97306180
(71) Applicant: SMITHKLINE BEECHAM PLC, Brentford, Middlesex TW8 9EP (GB)
(72) Inventor: Michalovich, David, SmithKline Beecham Pharm., Harlow, Essex CM19 5AW (GB)
(74) Representative: Crump, Julian Richard John

(57) **Abstract**

The use of U623 17 polypeptides and polynucleotides in the design of protocols for the treatment of cancer, developmental disorders, myeloid leukemia, immune disorders and neurodegenerative diseases, among others, and diagnostic assays for such conditions. Also disclosed are methods for producing such polypeptides by recombinant techniques.

## Description

### FIELD OF INVENTION

This invention relates to newly identified uses of polypeptides and the polynucleotides encoding for such, and to their production. More particularly, the polynucleotides and polypeptides of the present invention relate to JNK binding domain family, hereinafter referred to as U62317. The invention also relates to inhibiting or activating the action of such polynucleotides and polypeptides.

### BACKGROUND OF THE INVENTION

The c-Jun amino-terminal kinase (JNK) signal transduction pathway is activated in response to enviromental stress and activation of a variety of cell surface receptors such as cytokine receptors and receptor tyrosine kinases (reviewed by A.J. Whitmarsh and R.J. Davis, J.Mol.Med. 74:589, 1996). This pathway has been implicated in a number of processes including embryonic development, immune response, oncogenic transformation and programmed cell death. JNK regulates gene expression via target proteins such as c-Jun, activating transcription factor-2 and Elk-1. Gene activation via JNK requires entry of JNK into the nucleus. Recently a JNK interacting protein, JIP-1, has been identified in the mouse (M. Dickens et al., Science, 277:693-696, 1997). JIP-1 contains a JNK binding domain and a putative SRC homology 3 (SH3) domain and exhibits a widespread tissue expression pattern in the mouse, including brain, kidney, testis, lung and muscle. JTP-1 has been show to inhibit JNK mediated gene activation by retaining JNK in the cytoplasm, furthermore the JNK binding domain of JIP-1 has been shown to inhibit Bcr-Abl oncogenic transformation of pre-B lymphocytes, indicating JIP-1 to be potential therapeutic target (M. Dickens et al., Science, 277:693-696, 1997).

The GenBank database contains the complete sequence of a chromosome 22q13 BAC (bacterial artificial chromosome) clone CIT987SK-384D8; M.D. Adams et al. (1996) unpublished; GenBank Accession No. U62317. No function or potential therapeutic utility is given for this gene.

### SUMMARY OF THE INVENTION

In one aspect, the invention relates to U62317 polypeptides and recombinant materials and methods for their production. Another aspect of the invention relates to methods for using such U62317 polypeptides, polynucleotides and recombinant materials. Such uses include the treatment of cancer, developmental disorders, myeloid leukemia, immune disorders and neurodegenerative diseases, among others. In still another aspect, the invention relates to methods to identify agonists and antagonists using the materials provided by the invention, and treating conditions associated with U62317 imbalance with the identified compounds. Yet another aspect of the invention relates to diagnostic assays for detecting diseases associated with inappropriate U62317 activity or levels.

### DESCRIPTION OF THE INVENTION

### Definitions

The following definitions are provided to facilitate understanding of certain terms used frequently herein.

"U62317" refers, among others, generally to a polypeptide having the amino acid sequence set forth in SEQ ID NO:2 or an allelic variant thereof.

"U62317 activity or U62317 polypeptide activity" or "biological activity of the U62317 or U62317 polypeptide" refers to the metabolic or physiologic function of said U62317 including similar activities or improved activities or these activities with decreased undesirable side-effects. Also included are antigenic and immunogenic activities of said U62317.

"U62317 gene" refers to a polynucleotide having the nucleotide sequence set forth in SEQ ID NO: 1 or allelic variants thereof and/or their complements.

"Antibodies" as used herein includes polyclonal and monoclonal antibodies, chimeric, single chain, and humanized antibodies, as well as Fab fragments, including the products of an Fab or other immunoglobulin expression library.

"Isolated" means altered "by the hand of man" from the natural state. If an "isolated" composition or substance occurs in nature, it has been changed or removed from its original environment, or both. For example, a polynucleotide or a polypeptide naturally present in a living animal is not "isolated," but the same polynucleotide or polypeptide separated from the coexisting materials of its natural state is "isolated", as the term is employed herein.

"Polynucleotide" generally refers to any polyribonucleotide or polydeoxribonucleotide, which may be unmodified RNA or DNA or modified RNA or DNA. "Polynucleotides" include, without limitation single- and double-stranded DNA, DNA that is a mixture of single- and double-stranded regions, single- and double-stranded RNA, and RNA that is mixture of single-and double-stranded regions, hybrid molecules comprising DNA and RNA that may be single-stranded or, more typically, double-stranded or a mixture of single- and double-stranded regions. In addition, "polynucleotide" refers to triple-stranded regions comprising RNA or DNA or both RNA and DNA. The term polynucleotide also includes DNAs or RNAs containing one or more modified bases and DNAs or RNAs with backbones modified for stability or for other reasons. "Modified" bases include, for example, tritylated bases and unusual bases such as inosine. A variety of modifications has been made to DNA and RNA; thus, "polynucleotide" embraces chemically, enzymatically or metabolically modified forms of polynucleotides as typically found in nature, as well as the chemical forms of DNA and RNA characteristic of viruses and cells. "Polynucleotide" also embraces relatively short polynucleotides, often referred to as oligonucleotides.

"Polypeptide" refers to any peptide or protein comprising two or more amino acids joined to each other by peptide bonds or modified peptide bonds, i.e., peptide isosteres. "Polypeptide" refers to both short chains, commonly referred to as peptides, oligopeptides or oligomers, and to longer chains, generally referred to as proteins. Polypeptides may contain amino acids other than the 20 gene-encoded amino acids. "Polypeptides" include amino acid sequences modified either by natural processes, such as posttranslational processing, or by chemical modification techniques which are well known in the art. Such modifications are well described in basic texts and in more detailed monographs, as well as in a voluminous research literature. Modifications can occur anywhere in a polypeptide, including the peptide backbone, the amino acid side-chains and the amino or carboxyl termini. It will be appreciated that the same type of modification may be present in the same or varying degrees at several sites in a given polypeptide. Also, a given polypeptide may contain many types of modifications. Polypeptides may be branched as a result of ubiquitination, and they may be cyclic, with or without branching. Cyclic, branched and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent crosslinks, formation of cystine, formation of pyroglutamate, formylation, gamma-carboxylation, glycosylation, GPI anchor formation, hydroxylation, iodination, methylation, myristoylation, oxidation, proteolytic processing, phosphorylation, prenylation, racemization, selenoylation, sulfation, transfer-RNA mediated addition of amino acids to proteins such as arginylation, and ubiquitination. See, for instance, PROTEINS - STRUCTURE AND MOLECULAR PROPERTIES, 2nd Ed., T. E. Creighton, W. H. Freeman and Company, New York, 1993 and Wold, F., Posttranslational Protein Modifications: Perspectives and Prospects, pgs. 1-12 in POSTTRANSLATIONAL COVALENT MODIFICATION OF PROTEINS, B. C. Johnson, Ed., Academic Press, New York, 1983; Seifter *et al.,* "Analysis for protein modifications and nonprotein cofactors", *Meth Enzymol* (1990) 182:626-646 and Rattan *et al.,* "Protein Synthesis: Posttranslational Modifications and Aging", *Ann NY Acad Sci* (1992) 663 :48-62.

"Variant" as the term is used herein, is a polynucleotide or polypeptide that differs from a reference polynucleotide or polypeptide respectively, but retains essential properties. A typical variant of a polynucleotide differs in nucleotide sequence from another, reference polynucleotide. Changes in the nucleotide sequence of the variant may or may not alter the amino acid sequence of a polypeptide encoded by the reference polynucleotide. Nucleotide changes may result in amino acid substitutions, additions, deletions, fusions and truncations in the polypeptide encoded by the reference sequence, as discussed below. A typical variant of a polypeptide differs in amino acid sequence from another, reference polypeptide. Generally, differences are limited so that the sequences of the reference polypeptide and the variant are closely similar overall and, in many regions, identical. A variant and reference polypeptide may differ in amino acid sequence by one or more substitutions, additions, deletions in any combination. A substituted or inserted amino acid residue may or may not be one encoded by the genetic code. A variant of a polynucleotide or polypeptide may be a naturally occurring such as an allelic variant, or it may be a variant that is not known to occur naturally. Non-naturally occurring variants of polynucleotides and polypeptides may be made by mutagenesis techniques or by direct synthesis.

"% identity", as known in the art, is a measure of the relationship between two polypeptide sequences or two polynucleotide sequences, as determined by comparing their sequences. In general, the two sequences to be compared are aligned to give a maximum correlation between the sequences. The alignment of the two sequences is examined and the number of positions giving an exact amino acid or nucleotide correspondence between the two sequences determined, divided by the total length of the alignment and multiplied by 100 to give a % identity figure. This % identity figure may be determined over the whole length of the sequences to be compared, which is particularly suitable for sequences of the same or very similar length and which are highly homologous, or over shorter defined lengths, which is more suitable for sequences of unequal length or which have a lower level of homology.

"% similarity", as known in the art, is a further measure of the relationship between two polypeptide sequences. The two sequences being compared are aligned to give maximum correlation between the sequences. The alignment of the two sequences is examined at each position and a score is determined according to the chemical and/or physical properties of the amino acids being compared at that position. Such chemical and/or physical properties include charge, size and hydrophobicity of the amino acid side chains.

Methods for comparing the identity and similarity of two or more sequences are well known in the art. Thus for instance, programs available in the Wisconsin Sequence Analysis Package, version 9.1 (Devereux J et al, Nucleic Acids Res, 12, 387-395, 1984, available from Genetics Computer Group, Madison, Wisconsin, USA), for example the programs BESTFIT and GAP, may be used to determine the % identity between two polynucleotides and the % identity and the % similarity between two polypeptide sequences. BESTFIT uses the "local homology" algorithm of Smith and Waterman (Advances in Applied Mathematics, 2, 482-489, 1981) and finds the best single region of similarity between two sequences. BESTFIT is more suited to comparing two polynucleotide or two polypeptide sequences which are dissimilar in length, the program assuming that the shorter sequence represents a portion of the longer. In comparison, GAP aligns two sequences, finding a "maximum similarity", according to the algorithm of Neddleman and Wunsch (J Mol Biol, 48, 443-453, 1970). GAP is more suited to comparing sequences which are approximately the same length and an alignment is expected over the entire length. Preferably, the parameters "Gap Weight" and "Length Weight" used in each program are 50 and 3, for polynucleotide sequences and 12 and 4 for polypeptide sequences, respectively. Preferably, % identities and similarities are determined when the two sequences being compared are optimally aligned.

Other programs for determining identity and/or similarity between sequences are also known in the art, for instance the BLAST family of programs (Altschul S F et al, J Mol Biol, 215, 403-410, 1990, Altschul S F et al, Nucleic Acids Res., 25:389-3402, 1997, available from the National Center for Biotechnology Information (NCBI), Bethesda, Maryland, USA and accessible through the home page of the NCBI at www.ncbi.nlm.nih.gov) and FASTA (Pearson W R and Lipman D J, Proc Nat Acad Sci USA, 85, 2444-2448,1988, available as part of the Wisconsin Sequence Analysis Package). Preferably, the BLOSUM62 amino acid substitution matrix (Henikoff S and HenikoffJ G, Proc. Nat. Acad Sci. USA, 89, 10915-10919, 1992) is used in polypeptide sequence comparisons including where nucleotide sequences are first translated into amino acid sequences before comparison.

Preferably, the program BESTFIT is used to determine the % identity of a query polynucleotide or a polypeptide sequence with respect to a polynucleotide or a polypeptide sequence of the present invention, the query and the reference sequence being optimally aligned and the parameters of the program set at the default value.

### Polypeptides of the Invention

In one aspect, the present invention relates to U62317 polypeptides (or U62317 proteins). The U62317 polypeptides include the polypeptide of SEQ ID NO:2; as well as polypeptides comprising the amino acid sequence of SEQ ID NO: 2; and polypeptides comprising the amino acid sequence which have at least 80% identity to that of SEQ ID NO:2 over its entire length, and still more preferably at least 90% identity, and even still more preferably at least 95% identity to SEQ ID NO: 2. Furthermore, those with at least 97-99% are highly preferred. Also included within U62317 polypeptides are polypeptides having the amino acid sequence which have at least 80% identity to the polypeptide having the amino acid sequence of SEQ ID NO:2 over its entire length, and still more preferably at least 90% identity, and still more preferably at least 95% identity to SEQ ID NO:2. Furthermore, those with at least 97-99% are highly preferred. Preferably U62317 polypeptide exhibit at least one biological activity of U62317.

The U62317 polypeptides may be in the form of the "mature" protein or may be a part of a larger protein such as a precursor or fusion protein. It is often advantageous to include an additional amino acid sequence which contains secretory or leader sequences, pro-sequences, sequences which aid in purification such as multiple histidine residues, or an additional sequence for stability during recombinant production.

Fragments of the U62317 polypeptides are also included in the invention. A fragment is a polypeptide having an amino acid sequence that entirely is the same as part, but not all, of the amino acid sequence of the aforementioned U62317 polypeptides. As with U62317 polypeptides, fragments may be "free-standing," or comprised within a larger polypeptide of which they form a part or region, most preferably as a single continuous region. Representative examples of polypeptide fragments of the invention, include, for example, fragments from about amino acid number 1-20, 21-40, 41-60, 61-80, 81-100, and 101 to the end of U62317 polypeptide. In this context "about" includes the particularly recited ranges larger or smaller by several, 5, 4, 3, 2 or 1 amino acid at either extreme or at both extremes.

Preferred fragments include, for example, truncation polypeptides having the amino acid sequence of U62317 polypeptides, except for deletion of a continuous series of residues that includes the amino terminus, or a continuous series of residues that includes the carboxyl terminus or deletion of two continuous series of residues, one including the amino terminus and one including the carboxyl terminus. Also preferred are fragments characterized by structural or functional attributes such as fragments that comprise alpha-helix and alpha-helix forming regions, beta-sheet and beta-sheet-forming regions, turn and turn-forming regions, coil and coil-forming regions, hydrophilic regions, hydrophobic regions, alpha amphipathic regions, beta amphipathic regions, flexible regions, surface-forming regions, substrate binding region, and high antigenic index regions. Other preferred fragments are biologically active fragments. Biologically active fragments are those that mediate U62317 activity, including those with a similar activity or an improved activity, or with a decreased undesirable activity. Also included are those that are antigenic or immunogenic in an animal, especially in a human.

Preferably, all of these polypeptide fragments retain the biological activity of the U62317, including antigenic activity. Variants of the defined sequence and fragments also form part of the present invention. Preferred variants are those that vary from the referents by conservative amino acid substitutions -- i.e., those that substitute a residue with another of like characteristics. Typical such substitutions are among Ala, Val, Leu and Ile; among Ser and Thr; among the acidic residues Asp and Glu; among Asn and Gln; and among the basic residues Lys and Arg; or aromatic residues Phe and Tyr. Particularly preferred are variants in which several, 5-10, 1-5, or 1-2 amino acids are substituted, deleted, or added in any combination.

The U62317 polypeptides of the invention can be prepared in any suitable manner. Such polypeptides include isolated naturally occurring polypeptides, recombinantly produced polypeptides, synthetically produced polypeptides, or polypeptides produced by a combination of these methods. Means for preparing such polypeptides are well understood in the art.

### Polynucleotides of the Invention

Another aspect of the invention relates to U62317 polynucleotides. U62317 polynucleotides include isolated polynucleotides which encode the U62317 polypeptides and fragments, and polynucleotides closely related thereto. More specifically, U62317 polynucleotide of the invention include a polynucleotide comprising the nucleotide sequence contained in SEQ ID NO:1 encoding a U62317 polypeptide of SEQ ID NO: 2, and polynucleotides having the particular sequence of SEQ ID NO:1. U62317 polynucleotides further include a polynucleotide comprising a nucleotide sequence that has at least 80% identity over its entire length to a nucleotide sequence encoding the U62317 polypeptide of SEQ ID NO:2, and a polynucleotide comprising a nucleotide sequence that is at least 80% identical to that of SEQ ID NO:1 over its entire length. In this regard, polynucleotides at least 90% identical are particularly preferred, and those with at least 95% are especially preferred. Furthermore, those with at least 97% are highly preferred and those with at least 98-99% are most highly preferred, with at least 99% being the most preferred. Also included under U62317 polynucleotides are a nucleotide sequence which has sufficient identity to a nucleotide sequence contained in SEQ ID NO:1 to hybridize under conditions useable for amplification or for use as a probe or marker. The invention also provides polynucleotides which are complementary to such U62317 polynucleotides.

U62317 of the invention is structurally related to other proteins of the JNK binding domain family. The cDNA sequence of SEQ ID NO:1 contains an open reading frame (nucleotide number 1 to 1794) encoding a polypeptide of 598 amino acids of SEQ ID NO:2. Amino acid sequence of SEQ ID NO:2 has about 53% identity (using BESTFIT) in 211 amino acid residues with mouse JIP-1 (M. Dickens, et al., Science 277:693-696, 1997). Nucleotide sequence of SEQ ID NO:1 has about 70% identity (using Smith-Waterman) in 1345 nucleotide residues with mouse JIP-1 (M. Dickens, et al., Science 277:693-696, 1997).

A U62317 polynucleotide may be obtained using standard cloning and screening, from a cDNA library derived from mRNA in cells of human brain using the expressed sequence tag (EST) analysis (Adams, M.D., *et al. Science* (1991) 252:1651-1656; Adams, M.D. *et al., Nature,* (1992) 355:632-634; Adams, M.D., *et al., Nature* (1995) 377 Supp:3-174). Polynucleotides of the invention can also be obtained from natural sources such as genomic DNA libraries or can be synthesized using well known and commercially available techniques.

The nucleotide sequence encoding U62317 polypeptide of SEQ ID NO:2 may be identical to the polypeptide encoding sequence of SEQ ID NO:1, or it may be a sequence, which as a result of the redundancy (degeneracy) of the genetic code, also encodes the polypeptide of SEQ ID NO:2.

When the polynucleotides of the invention are used for the recombinant production of U62317 polypeptide, the polynucleotide may include the coding sequence for the mature polypeptide or a fragment thereof, by itself; the coding sequence for the mature polypeptide or fragment in reading frame with other coding sequences, such as those encoding a leader or secretory sequence, a pre-, or pro- or prepro- protein sequence, or other fusion peptide portions. For example, a marker sequence which facilitates purification of the fused polypeptide can be encoded. In certain preferred embodiments of this aspect of the invention, the marker sequence is a hexa-histidine peptide, as provided in the pQE vector (Qiagen, Inc.) and described in Gentz *et al., Proc Natl Acad Sci USA* (1989) 86:821-824, or is an HA tag. The polynucleotide may also contain noncoding 5' and 3' sequences, such as transcribed, non-translated sequences, splicing and polyadenylation signals, ribosome binding sites and sequences that stabilize mRNA. Further preferred embodiments are polynucleotides encoding U62317 variants comprising the amino acid sequence of U62317 polypeptide of SEQ ID NO:2 in which several, 5-10, 1-5, 1-3, 1-2 or 1 amino acid residues are substituted, deleted or added, in any combination.

The present invention further relates to polynucleotides that hybridize to the herein above-described sequences. In this regard, the present invention especially relates to polynucleotides which hybridize under stringent conditions to the herein above-described polynucleotides. As herein used, the term "stringent conditions" means hybridization will occur only if there is at least 80%, and preferably at least 90%, and more preferably at least 95%, yet even more preferably 97-99% identity between the sequences.

Polynucleotides of the invention, which are identical or sufficiently identical to a nucleotide sequence contained in SEQ ID NO:1 or a fragment thereof, may be used as hybridization probes for cDNA and genomic DNA, to isolate full-length cDNAs and genomic clones encoding U62317 polypeptide and to isolate cDNA and genomic clones of other genes (including genes encoding homologs and orthologs from species other than human) that have a high sequence similarity to the U62317 gene. Such hybridization techniques are known to those of skill in the art. Typically these nucleotide sequences are 80% identical, preferably 90% identical, more preferably 95% identical to that of the referent. The probes generally will comprise at least 15 nucleotides. Preferably, such probes will have at least 30 nucleotides and may have at least 50 nucleotides. Particularly preferred probes will range between 30 and 50 nucleotides.

One embodiment is to obtain a polynucleotide encoding U62317 polypeptide, including homologs and orthologs from species other than human, and comprises the steps of screening an appropriate library under stringent hybridization conditions with a labeled probe having the SEQ ID NO: 1 or a fragment thereof, and isolating full-length cDNA and genomic clones containing said polynucleotide sequence. Such hybridization techniques are well known to those of skill in the art. Thus in another aspect, U62317 polynucleotides of the present invention further include a nucleotide sequence comprising a nucleotide sequence that hybridize under stringent condition to a nucleotide sequence having SEQ ID NO: 1 or a fragment thereof. Also included with U62317 polypeptides are polypeptide comprising amino acid sequence encoded by nucleotide sequence obtained by the above hybridization condition. Stringent hybridization conditions are as defined above or, alternatively, conditions under overnight incubation at 42°C in a solution comprising: 50% formamide, 5xSSC (150mM NaCI, 15mM trisodium citrate), 50 mM sodium phosphate (pH7.6), 5x Denhardt's solution, 10 % dextran sulfate, and 20 microgram/ml denatured, sheared salmon sperm DNA, followed by washing the filters in 0.1x SSC at about 65°C.

The polynucleotides and polypeptides of the present invention may be employed as research reagents and materials for discovery of treatments and diagnostics to animal and human disease.

### Vectors, Host Cells, Expression

The present invention also relates to vectors which comprise a polynucleotide or polynucleotides of the present invention, and host cells which are genetically engineered with vectors of the invention and to the production of polypeptides of the invention by recombinant techniques. Cell-free translation systems can also be employed to produce such proteins using RNAs derived from the DNA constructs of the present invention.

For recombinant production, host cells can be genetically engineered to incorporate expression systems or portions thereof for polynucleotides of the present invention. Introduction of polynucleotides into host cells can be effected by methods described in many standard laboratory manuals, such as Davis et aL, *BASIC METHODS IN MOLECULAR BIOLOGY*(1986) and Sambrook et al., *MOLECULAR CLONING: A LABORATORY MANUAL,* 2nd Ed., Cold Spring Harbor Laboratory Press, Cold Spring Harbor, N.Y. (1989) such as calcium phosphate transfection, DEAE-dextran mediated transfection, transvection, microinjection, cationic lipid-mediated transfection, electroporation, transduction, scrape loading, ballistic introduction or infection.

Representative examples of appropriate hosts include bacterial cells, such as *Streptococci, Staphylococci, E. coli, Streptonyces* and *Bacillus subtilis* cells; fungal cells, such as yeast cells and *Aspergillus* cells; insect cells such as *Drosophila* S2 and *Spodoptera* Sf9 cells; animal cells such as CHO, COS, HeLa, C127, 3T3, BHK, HEK 293 and Bowes melanoma cells; and plant cells.

A great variety of expression systems can be used. Such systems include, among others, chromosomal, episomal and virus-derived systems, e.g., vectors derived from bacterial plasmids, from bacteriophage, from transposons, from yeast episomes, from insertion elements, from yeast chromosomal elements, from viruses such as baculoviruses, papova viruses, such as SV40, vaccinia viruses, adenoviruses, fowl pox viruses, pseudorabies viruses and retroviruses, and vectors derived from combinations thereof, such as those derived from plasmid and bacteriophage genetic elements, such as cosmids and phagemids. The expression systems may contain control regions that regulate as well as engender expression. Generally, any system or vector suitable to maintain, propagate or express polynucleotides to produce a polypeptide in a host may be used. The appropriate nucleotide sequence may be inserted into an expression system by any of a variety of well-known and routine techniques, such as, for example, those set forth in Sambrook *et al., MOLECULAR CLONING, A LABORATORY MANUAL* (*supra*).

For secretion of the translated protein into the lumen of the endoplasmic reticulum, into the periplasmic space or into the extracellular environment, appropriate secretion signals may be incorporated into the desired polypeptide. These signals may be endogenous to the polypeptide or they may be heterologous signals.

If the U62317 polypeptide is to be expressed for use in screening assays, generally, it is preferred that the polypeptide be produced at the surface of the cell. In this event, the cells may be harvested prior to use in the screening assay. If U62317 polypeptide is secreted into the medium, the medium can be recovered in order to recover and purify the polypeptide; if produced intracellularly, the cells must first be lysed before the polypeptide is recovered. U62317 polypeptides can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography and lectin chromatography. Most preferably, high performance liquid chromatography is employed for purification. Well known techniques for refolding proteins may be employed to regenerate active conformation when the polypeptide is denatured during isolation and or purification.

### Diagnostic Assays

This invention also relates to the use of U62317 polynucleotides for use as diagnostic reagents. Detection of a mutated form of U62317 gene associated with a dysfunction will provide a diagnostic tool that can add to or define a diagnosis of a disease or susceptibility to a disease which results from under-expression, over-expression or altered expression of U62317. Individuals carrying mutations in the U62317 gene may be detected at the DNA level by a variety of techniques.

Nucleic acids for diagnosis may be obtained from a subject's cells, such as from blood, urine, saliva, tissue biopsy or autopsy material. The genomic DNA may be used directly for detection or may be amplified enzymatically by using PCR or other amplification techniques prior to analysis. RNA or cDNA may also be used in similar fashion. Deletions and insertions can be detected by a change in size of the amplified product in comparison to the normal genotype. Point mutations can be identified by hybridizing amplified DNA to labeled U62317 nucleotide sequences. Perfectly matched sequences can be distinguished from mismatched duplexes by RNase digestion or by differences in melting temperatures. DNA sequence differences may also be detected by alterations in electrophoretic mobility of DNA fragments in gels, with or without denaturing agents, or by direct DNA sequencing. See, e.g., Myers *et al., Science* (1985) 230:1242. Sequence changes at specific locations may also be revealed by nuclease protection assays, such as RNase and S1 protection or the chemical cleavage method. See Cotton *et al., Proc Natl Acad Sci USA* (1985) 85: 4397-4401. In another embodiment, an array of oligonucleotides probes comprising U62317 nucleotide sequence or fragments thereof can be constructed to conduct efficient screening of e.g., genetic mutations. Array technology methods are well known and have general applicability and can be used to address a variety of questions in molecular genetics including gene expression, genetic linkage, and genetic variability. (See for example: M.Chee *et al., Science,* Vol 274, pp 610-613 (1996)).

The diagnostic assays offer a process for diagnosing or determining a susceptibility to cancer,developmental disorders, myeloid leukemia, immune disorders and neurodegenerative diseases through detection of mutation in the U62317 gene by the methods described.

In addition, cancer, developmental disorders, myeloid leukemia, immune disorders and neurodegenerative diseases, can be diagnosed by methods comprising determining from a sample derived from a subject an abnormally decreased or increased level of U62317 polypeptide or U62317 mRNA. Decreased or increased expression can be measured at the RNA level using any of the methods well known in the art for the quantitation of polynucleotides, such as, for example, PCR, RT-PCR, RNase protection, Northern blotting and other hybridization methods. Assay techniques that can be used to determine levels of a protein, such as an U62317 polypeptide, in a sample derived from a host are well-known to those of skill in the art. Such assay methods include radioimmunoassays, competitive-binding assays, Western Blot analysis and ELISA assays.

Thus in another aspect, the present invention relates to a diagonostic kit for a disease or suspectability to a disease, particularly cancer,developmental disorders, myeloid leukemia, immune disorders and neurodegenerative diseases, which comprises:
(a) a U62317 polynucleotide, preferably the nucleotide sequence of SEQ ID NO: 1, or a fragment thereof ;
(b) a nucleotide sequence complementary to that of (a);
(c) a U62317 polypeptide, preferably the polypeptide of SEQ ID NO: 2, or a fragment thereof; or
(d) an antibody to a U62317 polypeptide, preferably to the polypeptide of SEQ ID NO: 2.
It will be appreciated that in any such kit, (a), (b), (c) or (d) may comprise a substantial component.

### Chromosome Assays

The nucleotide sequences of the present invention are also valuable for chromosome localisation. The sequence is specifically targeted to and can hybridize with a particular location on an individual human chromosome. The mapping of relevant sequences to chromosomes according to the present invention is an important first step in correlating those sequences with gene associated disease. Once a sequence has been mapped to a precise chromosomal location, the physical position of the sequence on the chromosome can be correlated with genetic map data. Such data are found, for example, in V. McKusick, Mendelian Inheritance in Man (available on line through Johns Hopkins University Welch Medical Library). The relationship between genes and diseases that have been mapped to the same chromosomal region are then identified through linkage analysis (coinheritance of physically adjacent genes). The differences in the cDNA or genomic sequence between affected and unaffected individuals can also be determined. If a mutation is observed in some or all of the affected individuals but not in any normal individuals, then the mutation is likely to be the causative agent of the disease.
The U62317 gene is located on human chromosome 22 between markers D22S1140 and D22S1160.

### Antibodies

The polypeptides of the invention or their fragments or analogs thereof, or cells expressing them can also be used as immunogens to produce antibodies immunospecific for the U62317 polypeptides. The term "immunospecific" means that the antibodies have substantiall greater affinity for the polypeptides of the invention than their affinity for other related polypeptides in the prior art.

Antibodies generated against the U62317 polypeptides can be obtained by administering the polypeptides or epitope-bearing fragments, analogs or cells to an animal, preferably a nonhuman, using routine protocols. For preparation of monoclonal antibodies, any technique which provides antibodies produced by continuous cell line cultures can be used. Examples include the hybridoma technique (Kohler, G. and Milstein, C., *Nature* (1975) 256:495-497), the trioma technique, the human B-cell hybridoma technique (Kozbor *et al., Immunology Today* (1983) 4:72) and the EBV-hybridoma technique (Cole *et al.,* MONOCLONAL ANTIBODIES AND CANCER THERAPY, pp. 77-96, Alan R. Liss, Inc., 1985).

Techniques for the production of single chain antibodies (U.S. Patent No. 4,946,778) can also be adapted to produce single chain antibodies to polypeptides of this invention. Also, transgenic mice, or other organisms including other mammals, may be used to express humanized antibodies.

The above-described antibodies may be employed to isolate or to identify clones expressing the polypeptide or to purify the polypeptides by affinity chromatography.

Antibodies against U62317 polypeptides may also be employed to treat cancer, developmental disorders, myeloid leukemia, immune disorders and neurodegenerative diseases, among others.

### Vaccines

Another aspect of the invention relates to a method for inducing an immunological response in a mammal which comprises inoculating the mammal with U62317 polypeptide, or a fragment thereof, adequate to produce antibody and/or T cell immune response to protect said animal from cancer,developmental disorders, myeloid leukemia, immune disorders and neurodegenerative diseases, among others. Yet another aspect of the invention relates to a method of inducing immunological response in a mammal which comprises, delivering U62317 polypeptide via a vector directing expression of U62317 polynucleotide *in vivo* in order to induce such an immunological response to produce antibody to protect said animal from diseases.

Further aspect of the invention relates to an immunological/vaccine formulation (composition) which, when introduced into a mammalian host, induces an immunological response in that mammal to a U62317 polypeptide wherein the composition comprises a U62317 polypeptide or U62317 gene. The vaccine formulation may further comprise a suitable carrier. Since U62317 polypeptide may be broken down in the stomach, it is preferably administered parenterally (including subcutaneous, intramuscular, intravenous, intradermal etc. injection). Formulations suitable for parenteral administration include aqueous and non-aqueous sterile injection solutions which may contain anti-oxidants, buffers, bacteriostats and solutes which render the formulation instonic with the blood of the recipient; and aqueous and non-aqueous sterile suspensions which may include suspending agents or thickening agents. The formulations may be presented in unit-dose or multi-dose containers, for example, sealed ampoules and vials and may be stored in a freeze-dried condition requiring only the addition of the sterile liquid carrier immediately prior to use. The vaccine formulation may also include adjuvant systems for enhancing the immunogenicity of the formulation, such as oil-in water systems and other systems known in the art. The dosage will depend on the specific activity of the vaccine and can be readily determined by routine experimentation.

### Screening Assays

The U62317 polypeptide of the present invention may be employed in a screening process for compounds which activate (agonists) or inhibit activation of (antagonists, or otherwise called inhibitors) the U62317 polypeptide of the present invention. Thus, polypeptides of the invention may also be used to assess identify agonist or antagonists from, for example, cells, cell-free preparations, chemical libraries, and natural product mixtures. These agonists or antagonists may be natural or modified substrates, ligands, enzymes, receptors, etc., as the case may be, of the polypeptide of the present invention; or may be structural or functional mimetics of the polypeptide of the present invention. See Coligan *et al., Current Protocols in Immunology* 1(2):Chapter 5 (1991).

U62317 polypeptides are responsible for one or more biological functions, including one or more pathologies. Accordingly, it is desirous to find compounds and drugs which stimulate U62317 polypeptide on the one hand and which can inhibit the function of U62317 polypeptide on the other hand. In general, agonists are employed for therapeutic and prophylactic purposes for such conditions as cancer, developmental disorders, myeloid leukemia, immune disorders and neurodegenerative diseases. Antagonists may be employed for a variety of therapeutic and prophylactic purposes for such Conditions as cancer, developmental disorders, myeloid leukemia, immune disorders and neurodegenerative diseases.

In general, such screening procedures may involve using appropriate cells which express the U62317 polypeptide or respond to U62317 polypeptide of the present invention. Such cells include cells from mammals, yeast, *Drosophila* or *E. coli.* Cells which express the U62317 polypeptide (or cell membrane containing the expressed polypeptide) or respond to U62317 polypeptide are then contacted with a test compound to observe binding, or stimulation or inhibition of a functional response. The ability of the cells which were contacted with the candidate compounds is compared with the same cells which were not contacted for U62317 activity.

The assays may simply test binding of a candidate compound wherein adherence to the cells bearing the U62317 polypeptide is detected by means of a label directly or indirectly associated with the candidate compound or in an assay involving competition with a labeled competitor. Further, these assays may test whether the candidate compound results in a signal generated by activation of the U62317 polypeptide, using detection systems appropriate to the cells bearing the U62317 polypeptide. Inhibitors of activation are generally assayed in the presence of a known agonist and the effect on activation by the agonist by the presence of the candidate compound is observed.

Further, the assays may simply comprise the steps of mixing a candidate compound with a solution containing a U62317 polypeptide to form a mixture, measuring U62317 activity in the mixture, and comparing the U62317 activity of the mixture to a standard.

The U62317 cDNA, protein and antibodies to the protein may also be used to configure assays for detecting the effect of added compounds on the production of U62317 mRNA and protein in cells. For example, an ELISA may be constructed for measuring secreted or cell associated levels of U62317 protein using monoclonal and polyclonal antibodies by standard methods known in the art, and this can be used to discover agents which may inhibit or enhance the production of U62317 (also called antagonist or agonist, respectively) from suitably manipulated cells or tissues.

The U62317 protein may be used to identify membrane bound or soluble receptors, if any, through standard receptor binding techniques known in the art. These include, but are not limited to, ligand binding and crosslinking assays in which the U62317 is labeled with a radioactive isotope (eg 125I), chemically modified (eg biotinylated), or fused to a peptide sequence suitable for detection or purification, and incubated with a source of the putative receptor (cells, cell membranes, cell supernatants, tissue extracts, bodily fluids). Other methods include biophysical techniques such as surface plasmon resonance and spectroscopy. In addition to being used for purification and cloning of the receptor, these binding assays can be used to identify agonists and antagonists of U62317 which compete with the binding of U62317 to its receptors, if any. Standard methods for conducting screening assays are well understood in the art.

Examples of potential U62317 polypeptide antagonists include antibodies or, in some cases, oligonucleotides or proteins which are closely related to the ligands, substrates, enzymes, receptors, etc., as the case may be, of the U62317 polypeptide, e.g., a fragment of the ligands, substrates, enzymes, receptors, etc.; or small molecules which bind to the polypeptide of the present invention but do not elicit a response, so that the activity of the polypeptide is prevented.

Thus in another aspect, the present invention relates to a screening kit for identifying agonists, antagonists, ligands, receptors, substrates, enzymes, etc. for U62317 polypeptides; or compounds which decrease or enhance the production of U62317 polypeptides, which comprises:
(a) a U62317 polypeptide, preferably that of SEQ ID NO:2;
(b) a recombinant cell expressing a U62317 polypeptide, preferably that of SEQ ID NO:2;
(c) a cell membrane expressing a U62317 polypeptide; preferably that of SEQ ID NO: 2; or
(d) antibody to a U62317 polypeptide, preferably that of SEQ ID NO: 2.
It will be appreciated that in any such kit, (a), (b), (c) or (d) may comprise a substantial component.

### Prophylactic and Therapeutic Methods

This invention provides methods of treating abnormal conditions such as, cancer,developmental disorders, myeloid leukemia, immune disorders and neurodegenerative diseases, related to both an excess of and insufficient amounts of U62317 polypeptide activity.

If the activity of U62317 polypeptide is in excess, several approaches are available. One approach comprises administering to a subject an inhibitor compound (antagonist) as hereinabove described along with a pharmaceutically acceptable carrier in an amount effective to inhibit the function of the U62317 polypeptide, such as, for example, by blocking the binding of ligands, substrates, enzymes, receptors, etc., or by inhibiting a second signal, and thereby alleviating the abnormal condition. In another approach, soluble forms of U62317 polypeptides still capable of binding the ligand, substrate, enzymes, receptors, etc. in competition with endogenous U62317 polypeptide may be administered. Typical embodiments of such competitors comprise fragments of the U62317 polypeptide.

In another approach, soluble forms of U62317 polypeptides still capable of binding the ligand in competition with endogenous U62317 polypeptide may be administered. Typical embodiments of such competitors comprise fragments of the U62317 polypeptide.

In still another approach, expression of the gene encoding endogenous U62317 polypeptide can be inhibited using expression blocking techniques. Known such techniques involve the use of antisense sequences, either internally generated or separately administered. See, for example, O'Connor, *JNeurochem* (1991) 56:560 in Oligodeoxynucleotides as Antisense Inhibitors of Gene Expression, CRC Press, Boca Raton, FL (1988). Alternatively, oligonucleotides which form triple helices with the gene can be supplied. See, for example, Lee *et al., Nucleic Acids Res* (1979) 6:3073; Cooney *et al., Science* (1988) 241:456; Dervan *et al., Science* (1991) 251:1360. These oligomers can be administered *per se* or the relevant oligomers can be expressed *in vivo.*

For treating abnormal conditions related to an under-expression of U62317 and its activity, several approaches are also available. One approach comprises administering to a subject a therapeutically effective amount of a compound which activates U62317 polypeptide, i.e., an agonist as described above, in combination with a pharmaceutically acceptable carrier, to thereby alleviate the abnormal condition. Alternatively, gene therapy may be employed to effect the endogenous production of U62317 by the relevant cells in the subject. For example, a polynucleotide of the invention may be engineered for expression in a replication defective retroviral vector, as discussed above. The retroviral expression construct may then be isolated and introduced into a packaging cell transduced with a retroviral plasmid vector containing RNA encoding a polypeptide of the present invention such that the packaging cell now produces infectious viral particles containing the gene of interest. These producer cells may be administered to a subject for engineering cells *in vivo* and expression of the polypeptide *in vivo.* For overview of gene therapy, see Chapter 20, *Gene Therapy and other Molecular Genetic-based Therapeutic Approaches,* (and references cited therein) in Human Molecular Genetics, T Strachan and A P Read, BIOS Scientific Publishers Ltd (1996). Another approach is to administer a therapeutic amount of U62317 polypeptides in combination with a suitable pharmaceutical carrier.

### Formulation and Administration

Peptides, such as the soluble form of U62317 polypeptides, and agonists and antagonist peptides or small molecules, may be formulated in combination with a suitable pharmaceutical carrier. Such formulations comprise a therapeutically effective amount of the polypeptide or compound, and a pharmaceutically acceptable carrier or excipient. Such carriers include but are not limited to, saline, buffered saline, dextrose, water, glycerol, ethanol, and combinations thereof. Formulation should suit the mode of administration, and is well within the skill of the art. The invention further relates to pharmaceutical packs and kits comprising one or more containers filled with one or more of the ingredients of the aforementioned compositions of the invention.

Polypeptides and other compounds of the present invention may be employed alone or in conjunction with other compounds, such as therapeutic compounds.

Preferred forms of systemic administration of the pharmaceutical compositions include injection, typically by intravenous injection. Other injection routes, such as subcutaneous, intramuscular, or intraperitoneal, can be used. Alternative means for systemic administration include transmucosal and transdermal administration using penetrants such as bile salts or fusidic acids or other detergents. In addition, if properly formulated in enteric or encapsulated formulations, oral administration may also be possible. Administration of these compounds may also be topical and/or localized, in the form of salves, pastes, gels and the like.

The dosage range required depends on the choice of peptide, the route of administration, the nature of the formulation, the nature of the subject's condition, and the judgment of the attending practitioner. Suitable dosages, however, are in the range of 0.1-100 µg/kg of subject. Wide variations in the needed dosage, however, are to be expected in view of the variety of compounds available and the differing efficiencies of various routes of administration. For example, oral administration would be expected to require higher dosages than administration by intravenous injection. Variations in these dosage levels can be adjusted using standard empirical routines for optimization, as is well understood in the art.

Polypeptides used in treatment can also be generated endogenously in the subject, in treatment modalities often referred to as "gene therapy" as described above. Thus, for example, cells from a subject may be engineered with a polynucleotide, such as a DNA or RNA, to encode a polypeptide *ex vivo,* and for example, by the use of a retroviral plasmid vector. The cells are then introduced into the subject.

All publications, including but not limited to patents and patent applications, cited in this specification are herein incorporated by reference as if each individual publication were specifically and individually indicated to be incorporated by reference herein as though fully set forth.

Sequence information
SEQ ID NO:1
1 ATGCT CCCCGATTT CCCTT CT CCT AGCACCT GGG CCCCT GGG CT ACT GCT GCCCT CT GGC
61 CCAG CCCT GCT AT CT CCCT CCGT CCT GCAGGACAG CCT CT CCCT GGGG CG CT CGGAG CAG
121 CCG CACCCCAT CT GCT CCTT CCAGGAT GACTT CCAGGAGT TT GAGAT GAT CGAT GACAAT
181 GAAGAGGAGGACGAT GAGGACGAGGAAGAGGAGGAGGAGGAGGAGGAGGGAGAT GGGGAA
241 GG CCAGGAGGGAGGAGACCCT GG CT CAGAGG CACCT G CCCCCGGG CCCCT T AT CCCCT CC
301 CCTT CCGT GGAGGAG CCCCACAAG CACCGG CCCACCACCCT CCGT CT GACCACACT GGGG
361 G CCCAGGACT CCCAGGACCCCGAGG CGG CCG CGGGG CCCGG CGG CGT GGAG CT GGT GGAC
421 AT GGAGACG CT CAACCCCACG CGT GACACCAT CACG CCG CT GT GGG CCG CG CCCGG CCG C
481 G CCG CCCG CCCGGGACGAG CCT G CT CCG CCG CCT G CT CCGAGGAGGAGGACGAAGAGGAC
541 GACGAGGAAGAGGAGGAT G CCGAGGA CAGT G CGGGGT CCCCCGGGGG CAGGGG CACGGG C
601 CCCT CGG CG CCG CGGGACG CGT CG CT GGT GT ACGACG CGGT CAAGT ACACG CT GGT GGT G
661 GAT GAG CACACG CAG CT GGAG CT GGT GAG CCT G CGG CG CT GT G CT GGG CT GGG CCACGAC
721 AG CGAAGAGGACAG CGG CGGGGAGG CCAG CGAGGAGGAGG CGGGG CG CGG CG CT G CT AGG C
781 GG CGGT CAGGT CT CGGGGGACACCT CG CCGGACAG CCCT GACCT CACTTT CT CCAAGAAG
841 TT CCT CAAT GT CTT CGT CAACAG CACAT CT CGGT CCT CCAG CACCGAGT CCTTT GG CCTT
901 TT CT CCT GT CT GGT CAACGG CGAGGAG CGAGAG CAGACT CACCGGG CT GT GTT CAGGTT C
961 AT CCCG CGG CAT CCAGACGAG CT GGAG CT GGAT GT GGAT GACCCT GT GTT GGT GGAGG CC
1021 GAGGAGGACGACTT CT GGTT CCGT GG CTT CAACAT G CG CACGGGGGAG CG CGGT GT GTTT
1081 CCT G CCTT CT ACG CCCAT G CGGT G CCCGG CCCT G CCAAGGACCT G CT GGGGAGT AAG CGG
1141 AG CCCCT G CT GGGT GGAG CG CTTT GACGT G CAGTT CCT GGG CT CCGT GGAGGT G CCCT G C
1201 CACCAGGG CAACGG CAT CCT GT GT G CAG CCAT G CAGAAGATT G CCACT G CCCGGAAACT G
1261 ACCGT CCACCT G CG CCCT CCT G CCT CCT GT GACCT CGAGAT CT CT CTT CGGGGGGT CAAG
1321 CT GAGT CT GAG CGGAGGAGGG CCCGAGTT CCAG CG CT G CAG CCATTT CTT CCAGAT GAAG
1381 AACAT CT CCTT CT G CGG CT G CCAT CCCCG CAACAG CT G CGAGG CCCCG CAAGGAG CT G CT
1441 TT CCAGT GGGAG CGAGGGGT AGACCGGAAGAGGGTTTT ACAGACCAGGGGT AACGT CCAG
1501 CCCCACCT CGGAG CT GGG CAAGGGG CT G CCCT GAAT AGGG CCACAGAGGGT AG CAG CACC
1561 GG CT CAGAGAAGGGAGAGT GGACG CCCCT GGT CAT CAT GGAATT AACT CAGAG CGT GAAC
1621 T CTT G CT ATTT CGG CTT CAT CACCAAACACCCCCT G CT GAG CCG CTT CG CCT G CCACGT C
1681 TTT GT CT CCCAGGAGT CCAT GAGG CCGGT GG CG CAGAGT GT GGG CCG CG CCTT CCT GGAG
1741 T ACT ACCAAGAG CACCT GG CGT ACG CCT G CCCCACGGAGGACAT CT ACCT GGAGT AG CCT
1801 G CCCACCG CT CT GT CT CCT GG CCGT CT G CT CCAGG CG CAG CT GGGG CT GAGGAT GT CT CA
1861 AGAGG CCACCAT GG CTTT GG CAAGGACT GGATT GGGGGGACAT GGGACCTT ACG CTT GT G
1921 GGGGT CT G CGGG CT GGGAACT CT CGT CCT CGGT CCCCAGGG CG CAG CT GTT GGGG CT G CG
1981 GGGGAGT GGAG CCCCCGT G CCCCT G CTTTT CCT CAGAT CCGTT CTTT CT CT GT GTT GT CC
2041 T CCT CCTT CCCTT CCCAGT CT CCCTTTT CT CT CT CCT GGT GT CT CT G CCCCT CT CT GT G C
2101 CT G CAAACCTT AT CCT CT ATT CTT CACTTT GGGGT CAGAACTT CGGGGGT GT GGAGGAG
2161 T G CGG CT CT AGGGACAGGT AAT GT CGG CTT CCAAGT GAT G CCCT CCT G CCACT CCCCT G C
2221 GATTT AT AAGGGAATTTT AATTTTT AT AGT GAAT CT CAAGGGAT CAT CCCAT CCTT GACC
2281 ACAGGGACAAGAGGGG CCCCCT CG CCCCAG CCCCACCCCACAT GGAG CT CAGGGGGAAG C
2341 AGGGAGGGGTT CCCAAAAGAG CCCT G CGGAGG CT AGGAGT GGTT CTT GAT G CT CACCT GA

2401 AGCCCCTAGACGCTGCTAGGAGGGGGGCCCTCCTGGGCCCCCAATACCCCTCCGCTTGC
2461 CGCTGCTAGCGCACCCCTGCAGTCCAGCGTGCATCCACCCCACGCACGCCCCGTGCTGTG
2521 TTCCTGGGCCCTGCCCATGGCCTCAATAAACTCTCTGCTT

SEQ ID NO:2
1 MLPDFPSPSTWAPGLLLPSGPALLSPSVLQDSLSLGRSEQPHPICSFQDDFQEFEMIDDN
61 EEEDDEDEEEEEEEEEGDGEGQEGGDPGSEAPAPGPLIPSPSVEEPHKHRPTTLRLTTLG
121 AQDSQDPEAAAGPGGVELVDMETLNPTRDTITPLWAAPGRAARPGRACSAACSEEEDEED
181 DEEEEDAEDSAGSPGGRGTGPSAPRDASLVYDAVKYTLVVDEHTQLELVSLRRCAGLGHD
241 SEEDSGGEASEEEAGAALLGGGQVSGDTSPDSPDLTFSKKFLNVFVNSTSRSSSTESFGL
301 FSCLVNGEEREQTHRAVFRFIPRHPDELELDVDDPVLVEAEEDDFWFRGFNMRTGERGVF
361 PAFYAHAVPGPAKDLLGSKRSPCWVERFDVQFLGSVEVPCHQGNGILCAAMQKIATARKL
421 TVHLRPPASCDLEISLRGVKLSLSGGGPEFQRCSHFFQMKNISFCGCHPRNSCEAPQGAA
481 FQWERGVDRKRVLQTRGNVQPHLGAGQGAALNRATEGSSTGSEKGEWTPLVIMELTQSVN
541 SCYFGFITKHPLLSRFACHVFVSQESMRPVAQSVGRAFLEYYQEHLAYACPTEDIYLE

## Claims

1. A DNA or RNA molecule comprising an expression system, wherein said expression system is capable of producing a U623 17 polypeptide comprising an amino acid sequence, which has at least 80% identity with the polypeptide of SEQ ID NO:2 when said expression system is present in a compatible host cell.

2. A host cell comprising the expression system of claim 1.

3. A process for producing a U62317 polypeptide comprising culturing a host of claim 2 under conditions sufficient for the production of said polypeptide and recovering the polypeptide from the culture.

4. A process for producing a cell which produces a U62317 polypeptide thereof comprising transforming or transfecting a host cell with the expression system of claim 1 such that the host cell, under appropriate culture conditions, produces a U62317 polypeptide.

5. An antibody immunospecific for the U62317 polypeptide which comprises an amino acid sequence, which has at least 80% identity with the polypeptide of SEQ ID NO:2.

6. A method for the treatment of a subject in need of enhanced activity or expression of U62317 polypeptide comprising:
(a) administering to the subject a therapeutically effective amount of an agonist to said polypeptide; and/or
(b) providing to the subject an isolated polynucleotide comprising a nucleotide sequence that has at least 80% identity to a nucleotide sequence encoding the U62317 polypeptide of SEQ ID NO:2 over its entire length; or a nucleotide sequence complementary to said nucleotide sequence in a form so as to effect production of said polypeptide activity *in vivo.*

7. A method for the treatment of a subject having need to inhibit activity or expression of U62317 polypeptide comprising:
(a) administering to the subject a therapeutically effective amount of an antagonist to said polypeptide; and/or
(b) administering to the subject a nucleic acid molecule that inhibits the expression of the nucleotide sequence encoding said polypeptide; and/or
(c) administering to the subject a therapeutically effective amount of a polypeptide that competes with said polypeptide for its ligand, substrate, or receptor.

8. A process for diagnosing a disease or a susceptibility to a disease in a subject related to expression or activity of U62317 polypeptide of in a subject comprising:
(a) determining the presence or absence of a mutation in the nucleotide sequence encoding said U62317 polypeptide in the genome of said subject; and/or
(b) analyzing for the presence or amount of the U62317 polypeptide expression in a sample derived from said subject.

9. A method for identifying compounds which inhibit (antagonise) or agonise the U62317 polypeptide which comprises:
(a) contacting a candidate compound with cells which express the U62317 polypeptide (or cell membrane expressing U62317 polypeptide) or respond to U62317 polypeptide; and
(b) observing the binding, or stimulation or inhibition of a functional response; or comparing the ability of the cells (or cell membrane) which were contacted with the candidate compounds with the same cells which were not contacted for U62317 polypeptide activity.

10. An agonist or an antagonist identified by the method of claim 9.

11. A recombinant host cell produced by a method of Claim 4 or a membrane thereof expressing a U62317 polypeptide.


### Annex to the description

SEQUENCE LISTING

(1) GENERAL INFORMATION

(i) APPLICANT: SmithKline Beecham plc

(ii) TITLE OF THE INVENTION: Novel uses

(iii) NUMBER OF SEQUENCES: 2

(iv) CORRESPONDENCE ADDRESS:
(A) ADDRESSEE: SmithKline Beecham, Corporate Intellectual Property
(B) STREET: Two New Horizons Court
(C) CITY: Brentford
(D) STATE: Middlesex
(E) COUNTRY: UK
(F) ZIP: TW8 9EP

(v) COMPUTER READABLE FORM:
(A) MEDIUM TYPE: Diskette
(B) COMPUTER: IBM Compatible
(C) OPERATING SYSTEM: DOS
(D) SOFTWARE: FastSEQ for Windows Version 2.0

(2) INFORMATION FOR SEQ ID NO: 1:

(i) SEQUENCE CHARACTERISTICS:
(A) LENGTH: 2560 base pairs
(B) TYPE: nucleic acid
(C) STRANDEDNESS: single
(D) TOPOLOGY: linear

(ii) MOLECULE TYPE: cDNA

(xi) SEQUENCE DESCRIPTION: SEQ ID NO: 1:

ATGCTCCCCG ATTTCCCTTC TCCTAGCACC TGGGCCCCTG GGCTACTGCT GCCCTCTGGC 60

CCAGCCCTGC TATCTCCCTC CGTCCTGCAG GACAGCCTCT CCCTGGGGCG CTCGGAGCAG 120
CCGCACCCCA TCTGCTCCTT CCAGGATGAC TTCCAGGAGT TTGAGATGAT CGATGACAAT 180
GAAGAGGAGG ACGATGAGGA CGAGGAAGAG GAGGAGGAGG AGGAGGAGGG AGATGGGGAA 240
GGCCAGGAGG GAGGAGACCC TGGCTCAGAG GCACCTGCCC CCGGGCCCCT TATCCCCTCC 300
CCTTCCGTGG AGGAGCCCCA CAAGCACCGG CCCACCACCC TCCGTCTGAC CACACTGGGG 360
GCCCAGGACT CCCAGGACCC CGAGGCGGCC GCGGGGCCCG GCGGCGTGGA GCTGGTGGAC 420
ATGGAGACGC TCAACCCCAC GCGTGACACC ATCACGCCGC TGTGGGCCGC GCCCGGCCGC 480
GCCGCCCGCC CGGGACGAGC CTGCTCCGCC GCCTGCTCCG AGGAGGAGGA CGAAGAGGAC 540
GACGAGGAAG AGGAGGATGC CGAGGACAGT GCGGGGTCCC CCGGGGGCAG GGGCACGGGC 600
CCCTCGGCGC CGCGGGACGC GTCGCTGGTG TACGACGCGG TCAAGTACAC GCTGGTGGTG 660
GATGAGCACA CGCAGCTGGA GCTGGTGAGC CTGCGGCGCT GTGCTGGGCT GGGCCACGAC 720
AGCGAAGAGG ACAGCGGCGG GGAGGCCAGC GAGGAGGAGG CGGGCGCGGC GCTGCTAGGC 780
GGCGGTCAGG TCTCGGGGGA CACCTCGCCG GACAGCCCTG ACCTCACTTT CTCCAAGAAG 840
TTCCTCAATG TCTTCGTCAA CAGCACATCT CGGTCCTCCA GCACCGAGTC CTTTGGCCTT 900
TTCTCCTGTC TGGTCAACGG CGAGGAGCGA GAGCAGACTC ACCGGGCTGT GTTCAGGTTC 960
ATCCCGCGGC ATCCAGACGA GCTGGAGCTG GATGTGGATG ACCCTGTGTT GGTGGAGGCC 1020
GAGGAGGACG ACTTCTGGTT CCGTGGCTTC AACATGCGCA CGGGGGAGCG CGGTGTGTTT 1080
CCTGCCTTCT ACGCCCATGC GGTGCCCGGC CCTGCCAAGG ACCTGCTGGG GAGTAAGCGG 1140
AGCCCCTGCT GGGTGGAGCG CTTTGACGTG CAGTTCCTGG GCTCCGTGGA GGTGCCCTGC 1200
CACCAGGGCA ACGGCATCCT GTGTGCAGCC ATGCAGAAGA TTGCCACTGC CCGGAAACTG 1260
ACCGTCCACC TGCGCCCTCC TGCCTCCTGT GACCTCGAGA TCTCTCTTCG GGGGGTCAAG 1320
CTGAGTCTGA GCGGAGGAGG GCCCGAGTTC CAGCGCTGCA GCCATTTCTT CCAGATGAAG 1380
AACATCTCCT TCTGCGGCTG CCATCCCCGC AACAGCTGCG AGGCCCCGCA AGGAGCTGCT 1440
TTCCAGTGGG AGCGAGGGGT AGACCGGAAG AGGGTTTTAC AGACCAGGGG TAACGTCCAG 1500
CCCCACCTCG GAGCTGGGCA AGGGGCTGCC CTGAATAGGG CCACAGAGGG TAGCAGCACC 1560
GGCTCAGAGA AGGGAGAGTG GACGCCCCTG GTCATCATGG AATTAACTCA GAGCGTGAAC 1620
TCTTGCTATT TCGGCTTCAT CACCAAACAC CCCCTGCTGA GCCGCTTCGC CTGCCACGTC 1680
TTTGTCTCCC AGGAGTCCAT GAGGCCGGTG GCGCAGAGTG TGGGCCGCGC CTTCCTGGAG 1740
TACTACCAAG AGCACCTGGC GTACGCCTGC CCCACGGAGG ACATCTACCT GGAGTAGCCT 1800
GCCCACCGCT CTGTCTCCTG GCCGTCTGCT CCAGGCGCAG CTGGGGCTGA GGATGTCTCA 1860
AGAGGCCACC ATGGCTTTGG CAAGGACTGG ATTGGGGGGA CATGGACCTT TACGCTTGTG 1920
GGGGTCTGCG GGCTGGGAAC TCTCGTCCTC GGTCCCCAGG GCGCAGCTGT TGGGGCTGCG 1980
GGGGAGTGGA GCCCCCGTGC CCCTGCTTTT CCTCAGATCC GTTCTTTCTC TGTGTTGTCC 2040
TCCTCCTTCC CTTCCCAGTC TCCCTTTTCT CTCTCCTGGT GTCTCTGCCC CTCTCTGTGC 2100
CTGCAAACCT TATCCTCTAT TCTTCACTTT GGGGTCAGAA CTTCGGGGGT GTGGAGGAGG 2160
TGCGGCTCTA GGGACAGGTA ATGTCGGCTT CCAAGTGATG CCCTCCTGCC ACTCCCCTGC 2220
GATTTATAAG GGAATTTTAA TTTTTATAGT GAATCTCAAG GGATCATCCC ATCCTTGACC 2280
ACAGGGACAA GAGGGGCCCC CTCGCCCCAG CCCCACCCCA CATGGAGCTC AGGGGGAAGC 2340
AGGGAGGGGT TCCCAAAAGA GCCCTGCGGA GGCTAGGAGT GGTTCTTGAT GCTCACCTGA 2400
AGCCCCTAGA CGCTGCTAGG AGGGGGGGCC CTCCTGGGCC CCCAATACCC CTCCGCTTGC 2460
CGCTGCTAGC GCACCCCTGC AGTCCAGCGT GCATCCACCC CACGCACGCC CCGTGCTGTG 2520

TTCCTGGGCC CTGCCCATGG CCTCAATAAA CTCTCTGCTT                                    2560

(2) INFORMATION FOR SEQ ID NO: 2:

(i) SEQUENCE CHARACTERISTICS:
(A) LENGTH: 598 amino acids
(B) TYPE: amino acid
(C) STRANDEDNESS: single
(D) TOPOLOGY: linear

(ii) MOLECULE TYPE: protein

(xi) SEQUENCE DESCRIPTION: SEQ ID NO: 2:

Met Leu Pro Asp Phe Pro Ser Pro Ser Thr Trp Ala Pro Gly Leu Leu
1               5                   10                  15
Leu Pro Ser Gly Pro Ala Leu Leu Ser Pro Ser Val Leu Gln Asp Ser
            20                  25                  30
Leu Ser Leu Gly Arg Ser Glu Gln Pro His Pro Ile Cys Ser Phe Gln
        35                  40                  45
Asp Asp Phe Gln Glu Phe Glu Met Ile Asp Asp Asn Glu Glu Glu Asp
    50                  55                  60
Asp Glu Asp Glu Glu Glu Glu Glu Glu Glu Glu Glu Gly Asp Gly Glu
65                  70                  75                  80
Gly Gln Glu Gly Gly Asp Pro Gly Ser Glu Ala Pro Ala Pro Gly Pro
                85                  90                  95
Leu Ile Pro Ser Pro Ser Val Glu Glu Pro His Lys His Arg Pro Thr
            100                 105                 110
Thr Leu Arg Leu Thr Thr Leu Gly Ala Gln Asp Ser Gln Asp Pro Glu
        115                 120                 125
Ala Ala Ala Gly Pro Gly Gly Val Glu Leu Val Asp Met Glu Thr Leu
    130                 135                 140
Asn Pro Thr Arg Asp Thr Ile Thr Pro Leu Trp Ala Ala Pro Gly Arg
145                 150                 155                 160
Ala Ala Arg Pro Gly Arg Ala Cys Ser Ala Ala Cys Ser Glu Glu Glu
                165                 170                 175
Asp Glu Glu Asp Asp Glu Glu Glu Glu Asp Ala Glu Asp Ser Ala Gly
            180                 185                 190
Ser Pro Gly Gly Arg Gly Thr Gly Pro Ser Ala Pro Arg Asp Ala Ser
        195                 200                 205
Leu Val Tyr Asp Ala Val Lys Tyr Thr Leu Val Val Asp Glu His Thr

210                 215                 220

Gln Leu Glu Leu Val Ser Leu Arg Arg Cys Ala Gly Leu Gly His Asp
225                 230                 235                 240

Ser Glu Glu Asp Ser Gly Gly Glu Ala Ser Glu Glu Glu Ala Gly Ala
                245                 250                 255

Ala Leu Leu Gly Gly Gly Gln Val Ser Gly Asp Thr Ser Pro Asp Ser
            260                 265                 270

Pro Asp Leu Thr Phe Ser Lys Lys Phe Leu Asn Val Phe Val Asn Ser
        275                 280                 285

Thr Ser Arg Ser Ser Ser Thr Glu Ser Phe Gly Leu Phe Ser Cys Leu
    290                 295                 300

Val Asn Gly Glu Glu Arg Glu Gln Thr His Arg Ala Val Phe Arg Phe
305                 310                 315                 320

Ile Pro Arg His Pro Asp Glu Leu Glu Leu Asp Val Asp Asp Pro Val
                325                 330                 335

Leu Val Glu Ala Glu Glu Asp Asp Phe Trp Phe Arg Gly Phe Asn Met
            340                 345                 350

Arg Thr Gly Glu Arg Gly Val Phe Pro Ala Phe Tyr Ala His Ala Val
        355                 360                 365

Pro Gly Pro Ala Lys Asp Leu Leu Gly Ser Lys Arg Ser Pro Cys Trp
    370                 375                 380

Val Glu Arg Phe Asp Val Gln Phe Leu Gly Ser Val Glu Val Pro Cys
385                 390                 395                 400

His Gln Gly Asn Gly Ile Leu Cys Ala Ala Met Gln Lys Ile Ala Thr
                405                 410                 415

Ala Arg Lys Leu Thr Val His Leu Arg Pro Pro Ala Ser Cys Asp Leu
            420                 425                 430

Glu Ile Ser Leu Arg Gly Val Lys Leu Ser Leu Ser Gly Gly Gly Pro
        435                 440                 445

Glu Phe Gln Arg Cys Ser His Phe Phe Gln Met Lys Asn Ile Ser Phe
    450                 455                 460

Cys Gly Cys His Pro Arg Asn Ser Cys Glu Ala Pro Gln Gly Ala Ala
465                 470                 475                 480

Phe Gln Trp Glu Arg Gly Val Asp Arg Lys Arg Val Leu Gln Thr Arg
                485                 490                 495

Gly Asn Val Gln Pro His Leu Gly Ala Gly Gln Gly Ala Ala Leu Asn
            500                 505                 510

Arg Ala Thr Glu Gly Ser Ser Thr Gly Ser Glu Lys Gly Glu Trp Thr
        515                 520                 525

Pro Leu Val Ile Met Glu Leu Thr Gln Ser Val Asn Ser Cys Tyr Phe
    530                 535                 540

Gly Phe Ile Thr Lys His Pro Leu Leu Ser Arg Phe Ala Cys His Val
545                 550                 555                 560
Phe Val Ser Gln Glu Ser Met Arg Pro Val Ala Gln Ser Val Gly Arg
                565                 570                 575
Ala Phe Leu Glu Tyr Tyr Gln Glu His Leu Ala Tyr Ala Cys Pro Thr
            580                 585                 590
Glu Asp Ile Tyr Leu Glu
        595